# EUROPEAN PATENT SPECIFICATION

(11) **EP 4 368 109 B1**
(45) Date of publication and mention of the grant of the patent: **26.02.2025**
(21) Application number: 22215346.2
(22) Date of filing: 21.12.2022
(51) Int. Cl.: A61B 6/03, A61B 6/00

(54) **METHOD FOR TRAINING A SCATTER CORRECTION MODEL FOR USE IN AN X-RAY IMAGING SYSTEM**
VERFAHREN ZUM TRAINIEREN EINES STREUUNGSKORREKTURMODELLS ZUR VERWENDUNG IN EINEM RÖNTGENBILDGEBUNGSSYSTEM
PROCÉDÉ D'APPRENTISSAGE D'UN MODÈLE DE CORRECTION DE DIFFUSION DESTINÉ À ÊTRE UTILISÉ DANS UN SYSTÈME D'IMAGERIE PAR RAYONS X

(30) Priority: 11.11.2022 GR 20220100932
(43) Date of publication of application: 15.05.2024
(73) Proprietor: Carl Zeiss Industrielle Messtechnik GmbH, 73447 Oberkochen (DE)
(72) Inventor: Tzoumas, Stratis, 82065 Baierbrunn (DE); Krenkel, Martin, 73430 Aalen (DE)
(74) Representative: Patentanwälte Bressel und Partner mbB

(56) References cited:
- US-A1- 2018 330 233
- US-A1- 2022 035 961

## Description

The invention relates to a computer-implemented method for training a scatter correction model for use in an X-ray imaging system, a data processing apparatus, and a computer-readable medium. The invention further relates to a method for correcting scatter in X-ray image data of an X-ray imaging system.

Non-invasive imaging technologies allow imaging of the internal structures or features of a patient/object to be obtained without performing an invasive procedure on the patient/object. In one particular method, such non-invasive imaging technologies rely on the differential transmission of X-rays through a target volume to acquire image data and to construct images or otherwise represent the observed internal features of the patient/object.

Such imaging technique, including X-ray computed tomography (CT) and Cone-beam CT (CBCT), may be subject to scatter-type artifacts, in which the linear path of a particle that is detected as part of the imaging process is diverted during its transmission from some origination point to the detector apparatus. This non-linear travel is referred to as "scatter" and may be due to a particle interacting with one or more other particles or structures on its path through the imaged volume. As a consequence of such scatter events, the scattered particle may result in a detection event at a location that does not correspond to the expected linear path, which may lead to image artifacts since such linear paths are presumed in the reconstruction processes. Such scatter-related artifacts, typically leading to streaking or cupping, can have a negative impact on the image quality, the diagnostic value, and/or further evaluation of the resulting image. The prior art describes solutions which try to mitigate this problem by means of beam stops arranged in the beam path or by means of scatter simulation using statistical methods, such as the Monte Carlo method. Further, machine learning methods have been proposed to remove the scatter component of the signal, in particular using artificial neural networks and deep learning techniques.

US 2018 / 0 330 233 A1 discloses a method for machine learning based scatter correction. The approach relates to the use of machine learning in convolution kernel design for scatter correction. In one aspect, a neural network is trained to replace or improve the convolution kernel used for scatter correction. The training data set may be generated probabilistically so that actual measurements are not employed.

US 2021 / 0 007 692 A1 describes a method for correcting scattered radiation in a computed tomography apparatus, wherein x-ray radiation emanating from an x-ray radiation source is divided into a plurality of partial beams by a grid structure such that irradiated regions and non-irradiated regions alternate, wherein a grid position of the grid structure is changed parallel to a detector surface. In a changed grid position, previously non-irradiated regions are irradiated and previously irradiated regions are not irradiated, wherein at least one radiograph of the test object is captured for each of the grid positions, wherein the radiographs captured at different grid positions are used to generate a bright field radiograph from the respectively irradiated regions and a dark field radiograph from the respectively non-irradiated regions and wherein a corrected radiograph is generated on the basis of the bright field radiograph and the dark field radiograph.

US 2022 / 0 035 961 A1 describes a system and a method for artifact reduction of computed tomography reconstruction leveraging artificial intelligence and a priori known model for the object of interest. Nondestructive evaluation (NDE) of objects can elucidate impacts of various process parameters and qualification of the object. Computed tomography (CT) enables rapid NDE and characterization of objects. However, CT presents challenges because of artifacts produced by standard reconstruction algorithms. Beam-hardening artifacts especially complicate and adversely impact the process of detecting defects. By leveraging computer-aided design (CAD) models, CT simulations, and a deep-neutral network high-quality CT reconstructions that are affected by noise and beam-hardening can be simulated and used to improve reconstructions. The systems and methods of the present disclosure can significantly improve the reconstruction quality, thereby enabling better detection of defects compared with the state of the art.

Jun-Yan Zhu et al., Unpaired Image-to-Image Translation using Cycle-Consistent Adversarial Networks, Computer Vision and Pattern Recognition (cs.CV), arXiv:1703.10593 [cs.CV], , 24. August 2020, describes the application of cycle-consistent generative adversarial networks.

The invention is based on the technical problem of improving a method for training a scatter correction model for use in an X-ray imaging system.

According to the invention, the technical problem is solved by a computer-implemented method having the features of claim 1, a data processing apparatus having the features of claim 9, and a computer-readable medium having the features of claim 10. Advantageous embodiments of the invention emerge from the dependent claims.

One of the key ideas of the invention is to use simulated X-ray image data, simulated in particular with and without scattered radiation, and adapt the simulated X-ray image data with scattered radiation such that an adapted set of simulated X-ray image data resembles a more realistic representation of experimentally acquired X-ray image data with scattered radiation. This is achieved by using a domain adaptation technique, which allows to adapt the data domain of at least the simulated X-ray image data with scattered radiation (source domain) to the more realistic data domain of the experimental X-ray image data with scattered radiation (target domain). After processing the simulated X-ray image data with scattered radiation with the domain adaptation technique, an adapted set of simulated X-ray image data with scattered radiation is used to train the scatter correction model.

In particular, a computer-implemented method for training a scatter correction model for use in an X-ray imaging system is proposed, comprising generating a set of simulated X-ray image data with and without scattered radiation, processing the image data with scattered radiation of the set of simulated X-ray image data using a domain adaptation technique to provide an adapted set of simulated X-ray image data, wherein a target domain of the domain adaptation technique represents a set of experimental X-ray image data with scattered radiation, training the scatter correction model at least with the adapted set of simulated X-ray image data, and providing the trained scatter correction model for application in the X-ray imaging system.

Further, a data processing apparatus is proposed, comprising means for carrying out the method according to any one of the embodiments described in this disclosure.

In addition, a computer-readable medium is proposed, comprising instructions which, when executed by a computer, cause the computer to carry out the method according to any one of the embodiments described in this disclosure.

The proposed method allows to create a large set of realistic training X-ray image data with and without scattered radiation without the need of a great number of experimental X-ray image data. This leads to a reduction of time, effort, and cost necessary to train the scatter correction model. In particular, this allows quick training of the scatter correction model specifically for new objects to be inspected by the X-ray imaging system.

Experimental or simulated X-ray image data comprises, in particular, X-ray images. These X-ray images resemble images of a projection of X-rays through an object as it would be captured by an X-ray detector. In particular, the X-ray image data can comprise sets of X-ray images which are captured or simulated for the same object, wherein each image of a set corresponds to a different projection direction (e.g. defined by rotational angles between 0 and 360 degrees).

The scatter correction model is, in particular, a machine learning model, for example a machine learning model comprising at least one neural network. The scatter correction model is, in particular, trained to correct X-ray image data with scattered correction, such that the component that is a result of the scattered radiation is removed from the X-ray image data, i.e. the scatter correction model outputs scatter corrected image data ("scatter correction"). During training, pairs of X-ray image data, with and without scattered radiation, are used for adapting the weights and/or parameters of the machine learning model. In particular, the X-ray image data with scattered radiation are used as input while the X-ray image data without scattered radiation are used as corresponding ground truth at the output. Alternatively, the scatter correction model is, in particular, trained to identify the scattered radiation in the X-ray image data ("scatter estimation") and outputting a signal or image data that represents the scattered radiation component of the X-ray image data, such that the scattered radiation can be subsequently removed from the X-ray image data. During training, pairs of X-ray image data with scattered radiation and image data resembling the scattered radiation signal component are used for adapting the weights and/or parameters of the machine learning model. In particular, the X-ray image data with scattered radiation are used as input while the X-ray image data resembling the scattered radiation signal component are used as corresponding ground truth at the output.

X-ray image data without scattered radiation does not include a signal originating from scattering effects, while X-ray image data with scattered radiation includes the signal originating from scattering effects. The simulated X-ray image data with scattered radiation may be simulated using methods such as Monte Carlo and/or machine learning and/or approximate methods based on convolutions to derive the scattered radiation component of the total signal.

The X-ray imaging system may be, for example, a computed tomography (CT) system used for inspection of workpieces. The X-ray imaging system may also be a part of a coordinate measuring machine. The X-ray imaging system may also be an X-ray microscope.

In particular, the method is executed for a particular object. The target domain for the domain adaptation technique then corresponds to experimental X-ray image data with scattered radiation acquired for that particular object. The method for a particular object may be, for example, the following: Computer-implemented method for training a scatter correction model for a particular object scanned using an X-ray imaging system, comprising acquiring experimental X-ray image data for the particular object (i.e. a set of X-ray projection images from the particular object) that include scattered radiation (dataset A), generating a set of simulated X-ray image data with and without scattered radiation, processing the image data with scattered radiation of the set of simulated X-ray image data using a domain adaptation technique to provide an adapted set of simulated X-ray image data, wherein the target domain dataset for the domain adaptation technique is the dataset A, training the scatter correction model at least with the adapted set of simulated X-ray image data, and providing the trained scatter correction model for the particular object for application in the X-ray imaging system.

In one embodiment, the method further comprises acquiring and/or receiving a set of experimental X-ray image data with scattered radiation, training an adaptation network to be used as basis of the domain adaptation technique using the set of experimental X-ray image data with scattered radiation and the set of simulated X-ray image data with scattered radiation, wherein the adaptation network is trained using the principles of a generative adversarial network (GAN), wherein the adaptation network is or comprises the generative network of the generative adversarial network. This allows to create an adaptation network which is able to adapt the simulated X-ray image data with scattered radiation such that it is shifted to the more realistic data domain of the experimentally acquired X-ray image data with scattered radiation. The GAN comprises a generative network (generator) and a discriminative network (discriminator). As part of the method, the generator can be trained to create adapted simulated X-ray image data with scattered radiation. As input, the generator receives simulated X-ray image data with scattered radiation, which can be simulated by one of the methods mentioned above. As output, the generator provides adapted simulated X-ray image data with scattered radiation. During training, the discriminator is trained to discriminate between adapted simulated X-ray image data with scattered radiation and (real) experimental X-ray image data with scattered radiation. In other words, the generator tries to fool the discriminator while the discriminator tries to find out if its input is simulated or not. During training of the GAN, the generator and the discriminator are adapted until an equilibrium state is reached, in which the discriminator cannot easily tell the difference between the data produced by the generator and experimental X-ray image data with scattered radiation. After this training, the generator is used for implementing the domain adaptation technique. It is then able to adapt the simulated X-ray image data with scattered radiation to the target data domain of the experimental X-ray image data with scattered radiation. In particular, during training of the GAN, a "cycle consistency loss" is used as core complex constraint (cycle-GAN).

When executed for a particular object, the method for a particular object would further comprise, for example, acquiring and/or receiving a set of experimental X-ray image data of the particular object with scattered radiation, training an adaptation network to be used as basis of the domain adaptation technique using the set of experimental X-ray image data with scattered radiation and the set of simulated X-ray image data with scattered radiation, wherein the adaptation network is trained using the principles of a generative adversarial network, wherein the adaptation network is or comprises the generative network of the generative adversarial network.

The scatter correction model is trained additionally using at least one set of experimental X-ray image data with and without scattered radiation. This allows to additionally use experimental training data for training the scatter correction model.

In one embodiment, at least part of the set of experimental X-ray image data with and without scattered radiation is acquired by capturing X-ray images of at least one object using a movable beam stop array between the X-ray source and the at least one object. This allows to create experimental X-ray image data with and without scattering. In particular, a grid structure is used as a beam stop. A method may be used which has been described in US 2021 / 0 007 692 A1. The method uses a grid structure, which partially blocks the X-ray radiation and which is positioned between the X-ray source and the object to be imaged. At least two images are captured. The images are captured each at different positions of the grid structure with respect to a direction parallel to the detector plane. The images each comprise a bright (i.e. directly illuminated) portion and a dark (i.e. blocked) portion. The bright portions and the dark portions are stitched together, which results in a complete bright image with scattered radiation and a complete dark image containing only the scattered radiation. The difference between the bright and dark images is a scatter-corrected image. The bright image and the scatter-corrected image can then be used as experimental X-ray image data with and without scattered radiation.

In one embodiment, the set of simulated X-ray image data is at least partially generated by using a Computer Aided Design (CAD) model of at least one object. This way, the scatter correction model can be specifically trained for an object with known properties (geometry, size, material etc.). Based on the properties of the object a projection of X-rays through the object can be calculated which results in simulated X-ray image data. If scatter effects are taken into account using, for example, Monte Carlo or convolutional methods, simulated X-ray image data with scattered radiation can be created using the CAD model. In particular, the CAD model is a CAD model of the particular object.

In one embodiment, the set of simulated X-ray image data is at least partially generated by using a volume based forward projection of at least one object. The volume based forward projection uses a first reconstruction of measured x-ray images as the input for the simulation. The simulation then works by calculating, which fractions of which volume elements (voxels) are mapped to the respective pixels on the detector, using the geometry description which is anyway known for the reconstruction. To simulate ideal (scatter free) data, the volume can be segmented in its different materials prior to the forward projection. To simulate non-ideal data, the forward projected thickness can be weighted by the effective spectrum to include effects of beam-hardening. To simulate scatter effects convolution-based approaches can be used with the simulated projection images. Alternatively, it is possible to include a local neighborhood in the forward projection step to incorporate the 3D scattering information and/or to use Monte-Carlo simulations on the volume data. The volume based forward projection may be advantageous in terms of calculation times or when no CAD model is available.

In one developing embodiment, the CAD model and/or the volume is spatially aligned based on a tomographic reconstruction of experimentally acquired X-ray image data of the at least one object, in order to generate simulated X-ray image data that are aligned with the experimentally acquired X-ray image data. The alignment makes the simulated X-ray images correspond better to the acquired X-ray images that need to be corrected for scattering, and therefore it enhances the functional quality of the scatter correction model. In order to align the CAD model with the reconstructed X-ray volume, the CAD model can be transformed into a template volume where the voxels that are contained within the closed CAD model surface take the value 1, and the voxels outside the closed CAD model surface take the value 0. A similarity metric is used to compare the similarity between the template volume and the reconstructed X-ray volume, e.g. mutual information. 3D transformations (3D translation, rotation and scaling) are iteratively applied to the template volume and the transformed template is compared to the reconstructed X-ray volume with respect to the similarity metric. The 3D transformation that is found to maximize the similarity metric is used to transform the CAD model for better aligning the simulated and experimental data. Alternatively, the 3D transformation can be obtained by determining a surface of the reconstructed x-ray volume and applying registration algorithms to align the surface determined from the volume to the surface of the CAD model.

In one embodiment, the scatter correction model is adapted to work with downscaled versions of the X-ray image data. This allows lowering the computational budget necessary for training and/or application. In particular, the downscaling refers to reducing the total number of picture elements (pixels) representing the image data. In particular, the X-ray image data that is to be scatter-corrected is downscaled before inputting it to the scatter correction model. After application of the scatter correction model, output image data is upscaled again to the original scale (i.e. the original total number of picture elements). In one embodiment, the output resembles the scattered radiation component. This scattered radiation component is then upscaled to the original image size and subtracted by the original X-ray image data.

In one embodiment, providing the trained scatter correction model comprises loading a representation of the trained scatter correction model into a memory of at least one X-ray imaging system. The at least one X-ray imaging system can then apply the trained scatter correction model to correct captured X-ray images.

The invention is explained in detail below on the basis of preferred exemplary embodiments with reference to the drawings. In the drawings:
- Fig. 1: shows a schematic depiction to illustrate an embodiment of the (computer-implemented) method for training a scatter correction model for use in an X-ray imaging system;
- Fig. 2: shows a schematic depiction to illustrate an implementation of the domain adaptation technique;
- Fig. 3: shows a schematic depiction to illustrate another embodiment of the (computer-implemented) method for training a scatter correction model for use in an X-ray imaging system;
- Fig. 4: shows a schematic depiction of an embodiment of the data processing apparatus.

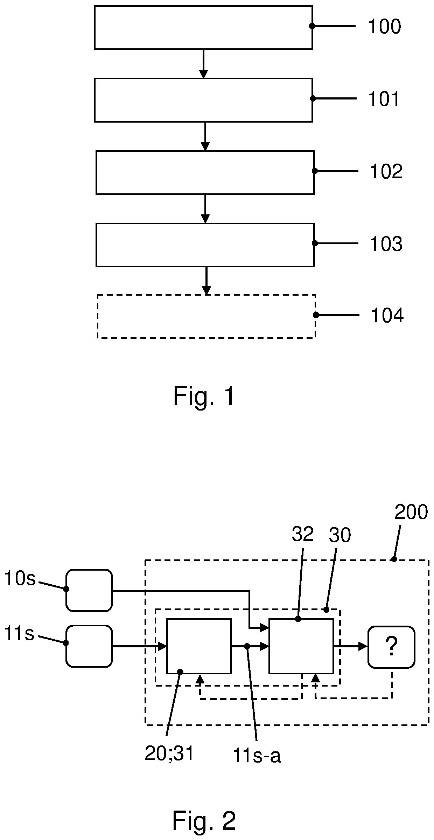

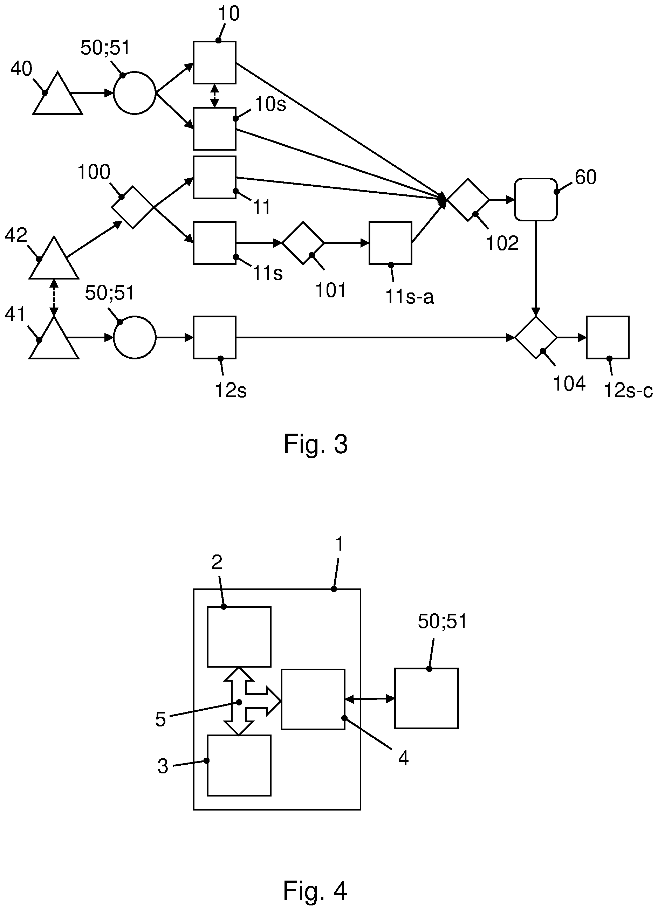

Fig. 1 shows an embodiment of the (computer-implemented) method for training a scatter correction model for use in an X-ray imaging system. The method is, in particular, executed on a data processing apparatus, such as the one shown in Fig. 4, for example.

Method step 100 comprises generating a set of simulated X-ray image data with and without scattered radiation. For example, CAD data of an object can be used to simulate the propagation and attenuation of X-rays through the volume body of the object and hence to calculate a signal projected onto a virtual detector area, which resembles the X-ray image data. The scattered radiation is included by calculating secondary radiation paths using, for example, Monte Carlo or convolutional methods. For each object simulated this way, at least one pair of X-ray images is provided, one X-ray image without scattered radiation and one with scattered radiation.

Method step 101 comprises processing the image data with scattered radiation of the set of simulated X-ray image data using a domain adaptation technique to provide an adapted set of simulated X-ray image data. A target domain of the domain adaptation technique represents a set of experimental X-ray image data with scattered radiation. In particular, the method is executed for a particular object. The target domain for the domain adaptation technique then corresponds to experimental X-ray image data with scattered radiation acquired for that particular object.

Method step 102 comprises training the scatter correction model at least with the adapted set of simulated X-ray image data. The scatter correction model is, in particular, a machine learning model. For example, a neural network, in particular a deep neural network, can be used as scatter correction model. During a training phase, the machine learning model, in particular the neural network, is trained by using at least the adapted set of simulated X-ray image data. The pairs of X-ray images without scattered radiation and with scattered radiation are used to adapt the parameters of the machine learning model by inputting the X-ray image with scattered radiation to the machine learning model, propagating it through the machine learning model, and comparing the output of the machine learning model with the corresponding X-ray image without the scattered radiation. In particular, the image without the scattered radiation is used as ground truth. Depending on the difference between the output of the machine learning model and the X-ray image without the scattered radiation the weights and/or parameters of the machine learning model are adapted. This is done for all the pairs of X-ray images in the set of adapted X-ray image data. The training cycles are repeated until the difference at the output is minimized below a desired value.

Method step 103 comprises providing the trained scatter correction model for application in the X-ray imaging system. This can comprise loading a representation of the trained scatter correction model into a memory of at least one X-ray imaging system, such that the trained scatter correction model can be applied to captured X-ray images of the X-ray imaging system.

Fig. 2 shows a schematic depiction to illustrate an implementation of the domain adaptation technique. In particular, Fig. 2 illustrates an embodiment for providing an adaptation network as part of the domain adaptation technique in method step 101. In the embodiment, the method further comprises acquiring and/or receiving a set of experimental X-ray image data 10s with scattered radiation (e.g. of a particular object), training an adaptation network 20 to be used as basis of the domain adaptation technique using the set of experimental X-ray image data 10s with scattered radiation and the set of simulated X-ray image data 11s with scattered radiation. The adaptation network 20 is trained in a training cycle 200 using the principles of a generative adversarial network 30 (GAN), wherein the adaptation network 20 is or comprises the generative network 31 of the generative adversarial network 30. The generative adversarial network 30 further comprises a discriminator network 32. In particular, the generative network 31 and the discriminator network 32 are and/or comprise neural networks. In particular, the generative network 31 is a U-Net. However, any fully-convolutional neural network architecture or a transformer architecture could be used for this task.

The principle of the training cycle 200 of the generative adversarial network 30, and the adaptation network 20 in particular, is described in the following. An X-ray image from the simulated X-ray image data 11s is given as an input to the adaptation network 20 (i.e. the generative network 31). The adaptation network 20 gives as output an adapted X-ray image 11s-a based on the initial weights and/or parameters of the adaptation network 20.

An X-ray image from the set of experimental X-ray image data 10s with scattered radiation and the adapted X-ray image 11s-a are given as input to the discriminator network 32, which tries to discriminate whether the images 10s, 11s (for simplicity, the reference numerals are used for the term image data as well as for the term image) provided are experimental ("real") or simulated ("fake"). The discriminator network 32 produces as output a prediction for both images 10s, 11s. The predictions are compared with the labels ("experimental" or "simulated") and the gradient is used to update the weights and/or parameters of the discriminator network 32, such that a prediction of the discriminator network 32 with the updated weights and/or parameters would be better. The inverse gradients of the discriminator network 32 are passed down to the generative network 31 and update the weights and/or parameters of the generative network 31, such that an image 11s adapted with the updated generative network 31 would cause the discriminator network 32 to make a worse prediction.

The training cycle 200 is repeated for further images 10s, 11s of the sets until a desired prediction accuracy of the discriminator network 32 is reached. Ideally, the prediction accuracy of the discriminator network 32 is reaching 50 % for both cases, i.e. the discriminator network 32 cannot tell anymore, whether the image is experimental or simulated.

It is possible to take further constraints into consideration. For example, one constraint may be the use of an L2 norm between the simulated X-ray image 11s given as input to the generative network 31 and the adapted X-ray image 11s-a. This L2 norm may be computed at each training step of the generative network 31 and the gradients from this norm can update the weights and/or parameters of the generative network 31 along with the gradients coming from the discriminator network 32.

In particular, a core complex constraint of the type "cycle-consistency loss" can be used, which is described in Jun-Yan Zhu et al., Unpaired Image-to-Image Translation using Cycle-Consistent Adversarial Networks, Computer Vision and Pattern Recognition (cs.CV), arXiv:1703.10593 [cs.CV], 24. August 2020.

After the training cycle 200 is completed the fully trained adaptation network 20 (i.e. the fully trained generative network 31) is used for the domain adaptation in method step 101. The trained adaptation network 20 is able to shift the images within the set of simulated X-ray image data to a more realistic target domain, such that the simulated X-ray image data with scattered radiation becomes more realistic. This way, improved training data can be provided for training the scatter correction model. In particular, the scatter correction model is trained by using pairs of simulated X-ray images without scattered radiation and simulated X-ray images with scattered radiation which have been adapted (or "refined") by the trained adaptation network 20.

It is possible that the scatter correction model is trained additionally using at least one set of experimental X-ray image data with and without scattered radiation.

It is possible that at least part of the set of experimental X-ray image data with and without scattered radiation is acquired by capturing X-ray images of at least one object using a movable beam stop array between the X-ray source and the at least one object. In particular, the method and grid structure described in US 2021 / 0 007 692 A1 may be used.

In method step 100, it is possible that the set of simulated X-ray image data is at least partially generated by using a CAD model of at least one object.

Additionally or alternatively it is possible that the set of simulated X-ray image data is at least partially generated by using a volume based forward projection of at least one object.

Further, it is possible that the CAD model and/or the volume is spatially aligned based on a tomographic reconstruction of experimentally acquired X-ray image data of the at least one object, in order to generate simulated X-ray image data that are aligned with the experimentally acquired X-ray image data. This can be executed by comparing the simulated X-ray image data with the experimentally acquired X-ray image data and iteratively changing the position and/or orientation of the CAD model until the simulated X-ray image data matches the experimentally acquired X-ray image data up to a required tolerance value.

It is possible that the scatter correction model is adapted to work with downscaled versions of the X-ray image data. In particular, X-ray images acquired with the X-ray imaging system are downscaled, i.e. a number of total picture elements (pixels) of the images is reduced, and the downscaled version is processed by the scatter correction model. After the processing the output of the scatter correction model, in particular the scattered radiation component, is upscaled again to to original scale, i.e. the original total number of picture elements.

It is possible that providing the trained scatter correction model in method step 103 comprises loading a representation of the trained scatter correction model into a memory of at least one X-ray imaging system.

Fig. 3 shows a schematic depiction to illustrate another embodiment of the method. The goal is to include a new object 41 (i.e. a particular object) to the functionality of the (trained) scatter correction model 60. For training the scatter correction model 60 objects 40 are scanned by an X-ray imaging system 50, in particular a computed tomography system 51, resulting in experimental X-ray image data 10 (stacks of images) without scattered radiation and corresponding experimental X-ray image data 10s (stacks of images) with scattered radiation. To obtain the X-ray image data with and without scattered radiation a method similar to the one described in US 2021 / 0 007 692 A1 may be used.

The new object 41 is described by a CAD model 42 of the new object 41. Using a simulation method of the prior art, the Monte Carlo method mentioned above, for example, simulated X-ray image data 11 (stack of images) without scattered radiation and simulated X-ray image data 11s (stack of images) with scattered radiation 11s are generated (method step 100).

The simulated X-ray image data 11s with scattered radiation is processed (method step 101) by the trained adaptation network 20 (i.e. the trained generative network 31, cf. Fig. 2), resulting in adapted simulated X-ray image data 11s-a.

The pairs of experimental X-ray image data 10, 10s with and without scattered radiation and pairs of the simulated X-ray image data 11 without scattered radiation and the adapted simulated X-ray image data 11s-a are used to train the scatter correction model 60, a deep neural network, for example (method step 102), resulting in a trained scatter correction model 60.

The trained scatter correction model 60 is then applied to captured experimental X-ray image data 12s (stack of images) with scattered radiation which are captured by the X-ray imaging system 50, in particular the computed tomography system 51, from the new object 41 in a normal mode, i.e. without the use of any hardware to correct or eliminate scattered radiation (method step 104). The application of the trained scatter correction model 60 results in scatter-corrected experimental X-ray image data 12s-c (stack of images) of the new object 41.

In particular, a method for correcting scatter in X-ray image data 12s of an X-ray imaging system 50 is thereby provided, wherein the trained scatter correction model 60 is used for correcting X-ray image data 12s, and wherein the scatter correction model 60 is or was trained using the method steps 100-104 (cf. also Fig. 1) described above.

Fig. 4 shows a schematic depiction of an embodiment of the data processing apparatus 1 which can be used to carry out the method according to any one of the embodiments described in this disclosure. The data processing apparatus 1 comprises a processing unit 2, which comprises at least one microprocessor (e.g. CPU, GPU etc.), a memory 3, an interface 4, and a communication bus 5. The processing unit 2, the memory 3, and the interface 4 communicate via the communication bus 5. The processing unit 2 executes program code stored in the memory 3 to carry out the method according to any one of the embodiments described in this disclosure, except for the measures that require the capture of experimental X-ray image data. The interface 4 allows data transfer of captured experimental X-ray image data from an X-ray imaging system 50, in particular from a computed tomography system 51, and to load the trained scatter correction model into a memory of the X-ray imaging system 50. Further the interface 4 allows controlling the data processing apparatus 1 and the method.

### List of reference numerals

- 1: data processing apparatus
- 2: processing unit
- 3: memory
- 4: interface
- 5: communication bus
- 10: experimental X-ray image data without scattered radiation
- 10s: experimental X-ray image data with scattered radiation
- 11: simulated X-ray image data without scattered radiation
- 11s: simulated X-ray image data with scattered radiation
- 11s-a: adapted simulated X-ray image data with scattered radiation
- 12s: experimental X-ray image data with scattered radiation
- 12c: corrected experimental X-ray image data
- 20: adaptation network
- 30: generative adversarial network
- 31: generative network
- 32: discriminator network
- 40: object(s)
- 41: new object
- 42: CAD model (of new object)
- 50: X-ray imaging system
- 51: computed tomography system
- 60: (trained) scatter correction model
- 100-104: method steps
- 200: training cycle (adaptation network)

## Claims

1. Computer-implemented method for training a scatter correction model (60) for use in an X-ray imaging system (50), comprising:
generating a set of simulated X-ray image data (11,11s) with and without scattered radiation,
processing the image data (11s) with scattered radiation of the set of simulated X-ray image data (11,11s) using a domain adaptation technique to provide an adapted set of simulated X-ray image data (11s-a), wherein a target domain of the domain adaptation technique represents a set of experimental X-ray image data (10s) with scattered radiation,
training the scatter correction model (60) at least with the adapted set of simulated X-ray image data (11s-a), and
providing the trained scatter correction model (60) for application in the X-ray imaging system (50),
wherein
the scatter correction model (60) is trained additionally using at least one set of experimental X-ray image data (10,10s) with and without scattered radiation.

2. Method according to claim 1, **characterized in that** the method further comprises acquiring and/or receiving a set of experimental X-ray image data (10s) with scattered radiation, training an adaptation network (20) to be used as basis of the domain adaptation technique using the set of experimental X-ray image data (10s) with scattered radiation and the set of simulated X-ray image data (11s) with scattered radiation, wherein the adaptation network (20) is trained using the principles of a generative adversarial network (30), wherein the adaptation network (20) is or comprises the generative network (31) of the generative adversarial network (30).

3. Method according to any one of the preceding claims, **characterized in that** at least part of the set of experimental X-ray image data (10,10s) with and without scattered radiation is acquired by capturing X-ray images of at least one object (40,41) using a movable beam stop array between the X-ray source and the at least one object (40,41).

4. Method according to any one of the preceding claims, **characterized in that**, the set of simulated X-ray image data (11,11s) is at least partially generated by using a Computer Aided Design, CAD, model (42) of at least one object (40,41).

5. Method according to any one of the preceding claims, **characterized in that**, the set of simulated X-ray image data (11,11s) is at least partially generated by using a volume based forward projection of at least one object (40,41).

6. Method according to any one of claims 4 or 5, **characterized in that** the CAD model (42) and/or the volume is spatially aligned based on a tomographic reconstruction of experimentally acquired X-ray image data (10,10s) of the at least one object, in order to generate simulated X-ray image data (11,11s) that are aligned with the experimentally acquired X-ray image data (10,11s).

7. Method according to any one of the preceding claims, **characterized in that** the scatter correction model (60) is adapted to work with downscaled versions of the X-ray image data (10,10s,11,11s).

8. Method according to any one of the preceding claims, **characterized in that** providing the trained scatter correction model (60) comprises loading a representation of the trained scatter correction model (60) into a memory of at least one X-ray imaging system (50).

9. A data processing apparatus (1) comprising a computer configured to carry out the method according to any one of the preceding claims.

10. A computer-readable medium, comprising instructions which, when executed by a computer, cause the computer to carry out the method according to any one of the claims 1 to 8.

11. Method for correcting scatter in X-ray image data (11s) of an X-ray imaging system (50), wherein a trained scatter correction model (60) is used for correcting X-ray image data (10s), wherein the scatter correction model (60) is or was trained using a method according to any one of the claims 1 to 8.

## Patentansprüche

1. Computerimplementiertes Verfahren zum Trainieren eines Streuungskorrekturmodells (60) zur Verwendung in einem Röntgenbildgebungssystem (50), umfassend:
Erzeugen eines Satzes simulierter Röntgenbilddaten (11, 11s) mit und ohne Streustrahlung,
Verarbeiten der Bilddaten (11s) mit Streustrahlung des Satzes simulierter Röntgenbilddaten (11, 11s) unter Verwendung einer Domänenadaptionstechnik, um einen angepassten Satz simulierter Röntgenbilddaten (11s-a) bereitzustellen, wobei eine Zieldomäne der Domänenadaptionstechnik einen Satz experimenteller Röntgenbilddaten (10s) mit Streustrahlung darstellt,
Trainieren des Streuungskorrekturmodells (60) zumindest mit dem angepassten Satz simulierter Röntgenbilddaten (11s-a) und
Bereitstellen des trainierten Streuungskorrekturmodells (60) zur Anwendung in dem Röntgenbildgebungssystem (50), wobei das Streuungskorrekturmodell (60) zusätzlich unter Verwendung mindestens eines Satzes experimenteller Röntgenbilddaten (10, 10s) mit und ohne Streustrahlung trainiert wird.

2. Verfahren nach Anspruch 1, **dadurch gekennzeichnet, dass** das Verfahren ferner Erfassen und/oder Empfangen eines Satzes experimenteller Röntgenbilddaten (10s) mit Streustrahlung, Trainieren eines Anpassungsnetzwerks (20), das als Basis der Domänenadaptionstechnik verwendet werden soll, unter Verwendung des Satzes experimenteller Röntgenbilddaten (10s) mit Streustrahlung und des Satzes simulierter Röntgenbilddaten (11s) mit Streustrahlung umfasst, wobei das Anpassungsnetzwerk (20) unter Verwendung der Prinzipien eines generativen kontradiktorischen Netzwerks (30) trainiert wird, wobei das Anpassungsnetzwerk (20) das generative Netzwerk (31) des generativen kontradiktorischen Netzwerks (30) ist oder umfasst.

3. Verfahren nach einem der vorhergehenden Ansprüche, **dadurch gekennzeichnet, dass** zumindest ein Teil des Satzes experimenteller Röntgenbilddaten (10, 10s) mit und ohne Streustrahlung durch Aufnehmen von Röntgenbildern mindestens eines Objekts (40, 41) unter Verwendung eines beweglichen Strahlstopp-Arrays zwischen der Röntgenquelle und dem mindestens einen Objekt (40, 41) erfasst wird.

4. Verfahren nach einem der vorhergehenden Ansprüche, **dadurch gekennzeichnet, dass** der Satz simulierter Röntgenbilddaten (11, 11s) zumindest teilweise durch Verwenden eines Computer-Aided-Design-Modells, CAD-Modells, (42) mindestens eines Objekts (40, 41) erzeugt wird.

5. Verfahren nach einem der vorhergehenden Ansprüche, **dadurch gekennzeichnet, dass** der Satz simulierter Röntgenbilddaten (11, 11s) zumindest teilweise durch Verwenden einer volumenbasierten Vorwärtsprojektion mindestens eines Objekts (40, 41) erzeugt wird.

6. Verfahren nach einem der Ansprüche 4 oder 5, **dadurch gekennzeichnet, dass** das CAD-Modell (42) und/oder das Volumen basierend auf einer tomographischen Rekonstruktion experimentell erfasster Röntgenbilddaten (10, 10s) des mindestens einen Objekts räumlich ausgerichtet werden, um simulierte Röntgenbilddaten (11, 11s) zu erzeugen, die mit den experimentell erfassten Röntgenbilddaten (10, 11s) ausgerichtet sind.

7. Verfahren nach einem der vorhergehenden Ansprüche, **dadurch gekennzeichnet, dass** das Streuungskorrekturmodell (60) angepasst wird, um mit herunterskalierten Versionen der Röntgenbilddaten (10, 10s, 11, 11s) zu arbeiten.

8. Verfahren nach einem der vorhergehenden Ansprüche, **dadurch gekennzeichnet, dass** das Bereitstellen des trainierten Streuungskorrekturmodells (60) Laden einer Darstellung des trainierten Streuungskorrekturmodells (60) in einen Speicher mindestens eines Röntgenbildgebungssystems (50) umfasst.

9. Datenverarbeitungsvorrichtung (1), umfassend einen Computer, der dazu ausgelegt ist, das Verfahren nach einem der vorhergehenden Ansprüche durchzuführen.

10. Computerlesbares Medium, umfassend Anweisungen, die bei Ausführung durch einen Computer den Computer zum Durchführen eines Verfahrens nach einem der Ansprüche 1 bis 8 veranlassen.

11. Verfahren zum Korrigieren von Streuung in Röntgenbilddaten (11s) eines Röntgenbildgebungssystems (50), wobei ein trainiertes Streuungskorrekturmodell (60) zum Korrigieren von Röntgenbilddaten (10s) verwendet wird, wobei das Streuungskorrekturmodell (60) unter Verwendung eines Verfahrens nach einem der Ansprüche 1 bis 8 trainiert wird oder wurde.

## Revendications

1. Procédé mis en œuvre par ordinateur pour l'entraînement d'un modèle de correction de diffusion (60) destiné à être utilisé dans un système d'imagerie par rayons X (50), comprenant :
la génération d'un ensemble de données d'images de rayons X simulées (11, 11s) avec et sans rayonnement diffusé,
le traitement des données d'image (11s) avec rayonnement diffusé de l'ensemble de données **d'images** de rayons X simulées (11, 11s) à l'aide d'une technique d'adaptation de domaine pour fournir un ensemble adapté de données d'images de rayons X simulées (11s-a), un domaine cible de la technique d'adaptation de domaine représentant un ensemble de données d'images de rayons X expérimentales (10s) avec rayonnement diffusé,
l'entraînement du modèle de correction de diffusion (60) au moins avec l'ensemble adapté de données d'images de rayons X simulées (11s-a), et
la fourniture du modèle de correction de diffusion entraîné (60) pour application dans le système d'imagerie par rayons X (50),
le modèle de correction de diffusion (60) étant en outre entraîné à l'aide d'au moins un ensemble de données d'images de rayons X expérimentales (10, 10s) avec et sans rayonnement diffusé.

2. Procédé selon la revendication 1, **caractérisé en ce que** le procédé comprend en outre l'acquisition et/ou la réception d'un ensemble de données d'images de rayons X expérimentales (10s) avec rayonnement diffusé, l'entraînement d'un réseau d'adaptation (20) à utiliser comme base de la technique d'adaptation de domaine à l'aide de l'ensemble de données d'images de rayons X expérimentales (10s) avec rayonnement diffusé et de l'ensemble de données d'images de rayons X simulées (11s) avec rayonnement diffusé, le réseau d'adaptation (20) étant entrainé selon les principes d'un réseau antagoniste génératif (30), le réseau d'adaptation (20) étant ou comprenant le réseau génératif (31) du réseau antagoniste génératif (30).

3. Procédé selon l'une quelconque des revendications précédentes, **caractérisé en ce qu'**au moins une partie de l'ensemble de données d'images de rayons X expérimentales (10, 10s) avec et sans rayonnement diffusé est acquise en capturant des images de rayons X d'au moins un objet (40, 41) à l'aide d'un réseau d'arrêt de faisceau mobile entre la source de rayons X et l'au moins un objet (40, 41) .

4. Procédé selon l'une quelconque des revendications précédentes, **caractérisé en ce que** l'ensemble de données d'images de rayons X simulées (11, 11s) est au moins partiellement généré en utilisant un modèle (42) de conception assistée par ordinateur, CAO, d'au moins un objet (40, 41).

5. Procédé selon l'une quelconque des revendications précédentes, **caractérisé en ce que** l'ensemble de données d'images de rayons X simulées (11, 11s) est au moins partiellement généré en utilisant une projection avant basée sur le volume d'au moins un objet (40, 41).

6. Procédé selon l'une quelconque des revendications 4 ou 5, **caractérisé en ce que** le modèle CAO (42) et/ou le volume est aligné spatialement sur la base d'une reconstruction tomographique de données d'images de rayons X acquises expérimentalement (10, 10s) de l'au moins un objet, afin de générer des données d'images de rayons X simulées (11, 11s) qui sont alignées sur les données d'images de rayons X acquises expérimentalement (10, 11s).

7. Procédé selon l'une quelconque des revendications précédentes, **caractérisé en ce que** le modèle de correction de diffusion (60) est adapté pour fonctionner avec des versions à échelle réduite des données d'images de rayons X (10, 10s, 11, 11s).

8. Procédé selon l'une quelconque des revendications précédentes, **caractérisé en ce que** la fourniture du modèle de correction de diffusion entraîné (60) comprend le chargement d'une représentation du modèle de correction de diffusion entraîné (60) dans une mémoire d'au moins un système d'imagerie par rayons X (50).

9. Appareil de traitement de données (1) comprenant un ordinateur configuré pour mettre en œuvre le procédé selon l'une quelconque des revendications précédentes.

10. Support lisible par ordinateur, comprenant des instructions qui, lorsqu'elles sont exécutées par un ordinateur, amènent l'ordinateur à mettre en œuvre le procédé selon l'une quelconque des revendications 1 à 8.

11. Procédé de correction de diffusion dans des données d'images de rayons X (11s) d'un système d'imagerie par rayons X (50), un modèle de correction de diffusion entraîné (60) étant utilisé pour corriger les données d'images de rayons X (10s), le modèle de correction de diffusion (60) étant ou ayant été entraîné à l'aide d'un procédé selon l'une quelconque des revendications 1 à 8.
